# EUROPEAN PATENT APPLICATION

(11) **EP 1 433 414 A1**
(43) Date of publication of application: **30.06.2004**
(21) Application number: 03029299.9
(22) Date of filing: 22.12.2003
(51) Int. Cl.: A61B 3/028

(54) **Optometric apparatus**

(30) Priority: 26.12.2002 JP 2002378205
(71) Applicant: Nidek Co., Ltd., Gamagori-shi, Aichi-ken 443-0035 (JP)
(72) Inventor: Hosoi, Yoshinobu, Gamagori-shi Aichi-ken, 443-0035 (JP)
(74) Representative: Hofer, Dorothea, Dipl.-Phys.

(57) **Abstract**

An optometric apparatus (1) for subjectively examining a refractive power of an eye of an examinee is disclosed. The apparatus includes a pair of lens chamber units (2) each having a test window (4), one for a right eye of the examinee and the other for a left eye; rotary disks (11-16) in each of which a plurality of optical elements (110-160) are set, the disks being rotatably placed in each lens chamber unit and including first rotary disks (11-13) placed at positions nearer the eye and second rotary disks (14-16) placed at positions farther from the eye. In the apparatus, the optical elements (140-160) in the second rotary disks are larger in effective diameter than the optical elements (110-130) in the first rotary disks.

## Description

### BACKGROUND OF THE INVENTION

### 1. Field of the Invention

The present invention relates to an optometric apparatus for subjectively examining a refractive power of an eye of an examinee.

### 2. Description of Related Art

There is a subjective-type optometric apparatus including a pair of lens chamber units; one for a right eye of an examinee and the other for a left eye. In each lens chamber unit, there are placed a plurality of rotary disks, each holding a plurality of optical elements such as a spherical lens and a cylindrical lens.

In the optometric apparatus of this type, commercially available, each optical element has a diameter of 20 mm (an effective diameter of 19 mm). An angle of view (sight) of an examinee's eye when views through each optical element is at most 34° to 36° provided that a visual distance (VD) is 12 mm. It is to be noted that "VD" is a distance between an apex of cornea of the examinee's eye and a surface of an optical element (a spherical lens) facing the examinee's eye in a rotary disk placed at a nearest position to the examinee's eye. However, depending on the angle of view, the intervention of accommodation (so-called instrument myopia) caused by a tube effect may have a significant influence on examination results. It is therefore desirable to provide a larger (wider) view angle in order to accurately perform eye examinations with little influence from the intervention of accommodation.

### SUMMARY OF THE INVENTION

The present invention has been made in view of the above circumstances and has an object to overcome the above problems and to provide an optometric apparatus capable of more accurately performing examinations with little influence from intervention of accommodation.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the purpose of the invention, there is provided an optometric apparatus for subjectively examining a refractive power of an eye of an examinee, the apparatus including: a pair of lens chamber units each having a test window, one for a right eye of the examinee and the other for a left eye; rotary disks in each of which a plurality of optical elements are set, the disks being rotatably placed in each lens chamber unit and including first rotary disks placed at positions nearer the eye and second rotary disks placed at positions farther from the eye; wherein the optical elements in the second rotary disks are larger in effective diameter than the optical elements in the first rotary disks.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1 is a schematic external front view of a subjective-type optometric apparatus, viewed from an examiner;
Fig. 2 is a schematic sectional view of a part of a lens chamber unit for a left eye, viewed from above;
Fig. 3 is a schematic sectional view showing a rotation system of optical elements set in rotary disks;
Fig. 4 is a table showing an example of arrangement of the optical elements in each rotary disk;
Fig. 5 is a schematic sectional view showing a concave portion provided by the lens chamber units for allowing an examinee's nose to be positioned;
Fig. 6 is an explanatory view showing an example of an eccentric amount between a small-diameter rotary disk and a large-diameter rotary disk;
Fig. 7 is an explanatory view showing an example that the eccentric amount between the large-diameter rotary disk and the small-diameter rotary disk is increased to provide two points at which the peripheral edges of both disks overlap;
Fig. 8A is a sectional view taken along a line A-A in Fig. 7; and
Fig. 8B is a sectional view taken along a line B-B in Fig. 7.

### DETAILED DESCRIPTION OF THE PREFERRED EMBODIMENTS

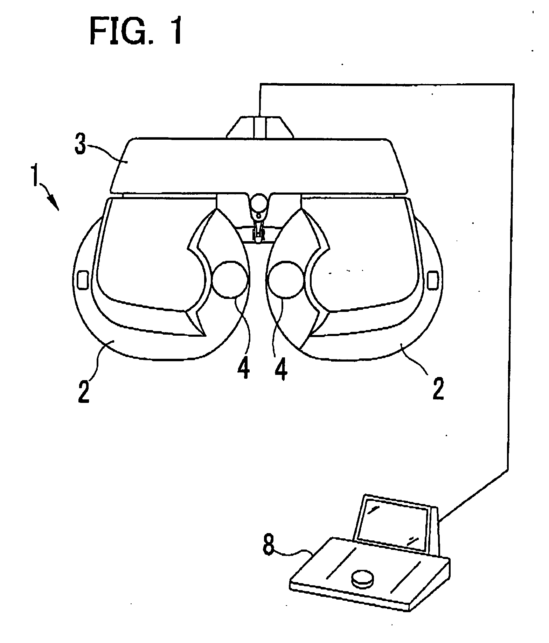

A detailed description of a preferred embodiment of an optometric apparatus embodying the present invention will now be given referring to the accompanying drawings. Fig. 1 is a schematic external front view of a subjective-type optometric apparatus in the present embodiment, viewed from an examiner.

A main unit 1 of the optometric apparatus is provided with a pair of lens chamber units 2 which are symmetrically constructed for a right and left eyes and a support unit 3 which supports (holds) the lens chamber units 2 in a suspended condition. In each lens chamber unit 2, a plurality of rotary disks are rotatably placed (held): On each rotary disk, plural optical elements such as a spherical lens and a cylindrical lens are set (held) at circumferentially spaced intervals. The optical elements in each disk are selectively disposed in test windows 4 provided in the lens chamber units 2 respectively. The support unit 3 includes, in order to change a distance (an interval) between the test windows 4 according to the pupillary distance of the examinee, a slide system for adjusting a distance (an interval) between the lens chamber units 2 and a convergence system for adjusting a convergence angle (an inward-turning angle) of the lens chamber units 2. These slide system and convergence system are well known, for example, which are disclosed in Japanese patent unexamined publication No. Hei 10(1998)-14872, USP 5,929,971, and DE 19728262Al. Those systems bear little relation to the invention and therefore they are not explained herein. It is to be noted that the main unit 1 is connected with an operating controller 8.

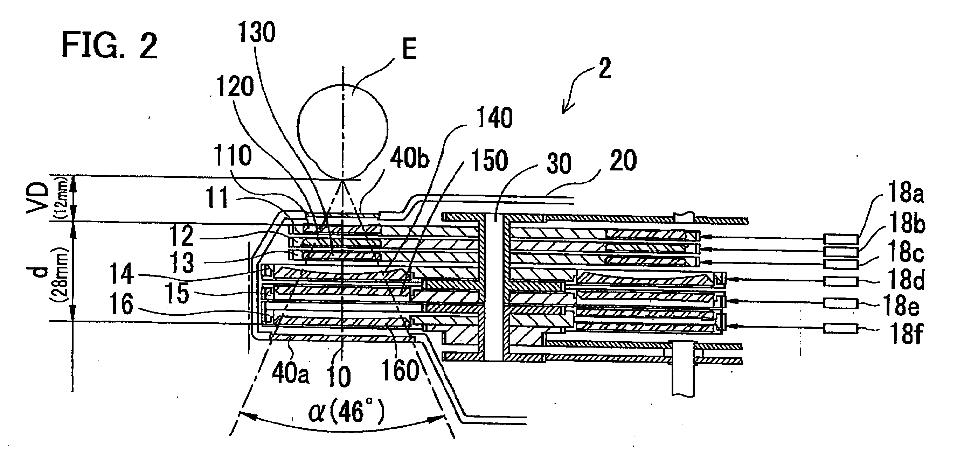

Fig. 2 is a schematic sectional view of a part of a lens chamber unit 2 for a left eye, viewed from above. Numeral 10 is an examination optical axis. "E" denotes the left eye of an examinee. Each lens chamber unit 2 includes a cover 20, in which six rotary disks 11-16 are placed (held) to be rotatable about a shaft 30. In each rotary disk 11-16, an opening and a plurality of optical elements are set (held). The rotary disks are a high-power spherical lens disk 11, a low-power spherical lens disk 12, a first auxiliary lens disk 13, a high-power cylindrical lens disk 14, a low-power cylindrical lens disk 15, and a second auxiliary lens disk 16, which are arranged in this order from nearer to farther with respect to the eye E. Each disk 11-16 has an outer periphery formed with gear teeth and is rotated by operation of a corresponding one of motors 18a-18f, thus changing an optical element to be disposed on the optical axis 10. It is to be noted that, in Fig. 2, numeral 40a is a protective glass fit in the test window 4 on an examiner side and 40b is a protective glass fit in the test window 4 on an examinee side.

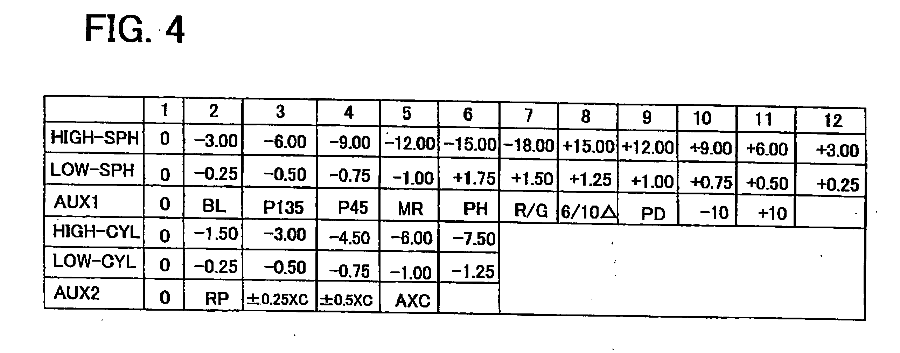

Fig. 4 is a table showing an example of arrangement of the optical elements in each rotary disk 11-16. Each disk 11-16 has at least one opening with no lens or a lens of 0D (diopter). In the present embodiment, the disks 11, 12, and 13 are about 110 mm in diameter and each disk 11-13 is formed with twelve holes in which optical elements are set (held).

The disk 11 holds eleven spherical lenses 110 of different high powers (refractive powers); -3D, -6D, -9D, -12D, -15D, -18D, +3D, +6D, +9D, +12D, and +15D. The disk 12 holds eleven spherical lenses 120 of different low powers (refractive powers); -0.25D, -0.5D, -0.75D, -1D, +0.25D, +0.5D, +0.75D, +1D, +1.25D, +1.5D, and +1.75D. The spherical lenses 110 and 120 in the disks 11 and 12 respectively are about 20 mm in diameter (19 mm in effective diameter).

The disk 13 holds a first group of auxiliary lenses 130 in the holes other than the opening. The auxiliary lenses 130 in the present embodiment are a shielding plate (BL), a polarizing plate (P135, P45), a Maddox lens (MR), a pinhole (PH), a red/green filter (R/G), a dispersion prism (6/10Δ), a noncorrecting lens (PD) with a mark for adjustment of an interpupillary distance (the interval between the right and left lens chamber units 2) according to the examinee's pupillary distance, a spherical lens of +10D, and a spherical lens of -10D. Each lens 130 in the disk 13 is about 20 mm in diameter (19 mm in effective diameter).

The disks 14, 15, and 16 in the present embodiment each have six holes in which optical elements are set (held). Each disk 14-16 is about 125 mm in diameter, which is larger than the disks 11, 12, and 13. In addition, the diameters (the effective diameters) of the optical elements in the disks 14, 15, and 16 are larger than those of the optical elements in the disks 11, 12, and 13.

The disk 14 holds five cylindrical lenses 140 of different high powers (refractive powers); -1.5D, -3D, -4.5D, -6D, -7.5D. The disk 15 holds five cylindrical lenses 150 of different low powers (refractive powers); -0.25D, -0.5D, -0.75D, -1D, -1.25D. These cylindrical lenses 140 and 150 in the disks 14 and 15 are about 36 mm in diameter (about 35 mm in effective diameter). The cylindrical lenses 140 and 150 are both set to be rotatable about the optical axis 10.

The disk 16 holds a second group of auxiliary lenses 160 in the holes other than the opening. The auxiliary lenses 160 in the present embodiment are two cross-cylinder lenses (XC) of ±0.25D and ±0.5D, an auto-cross-cylinder lens (AXC), a rotary prism (RP). As with the lenses 140 and 150, the lenses 160 in the disk 16 is about 36 mm in diameter (about 35 mm in effective diameter). The rotary prism and the cross-cylinder lens are both set to be rotatable about the optical axis 10. The rotary prism is preferably a Fresnel prism, which needs no increase in thickness of the disk 16.

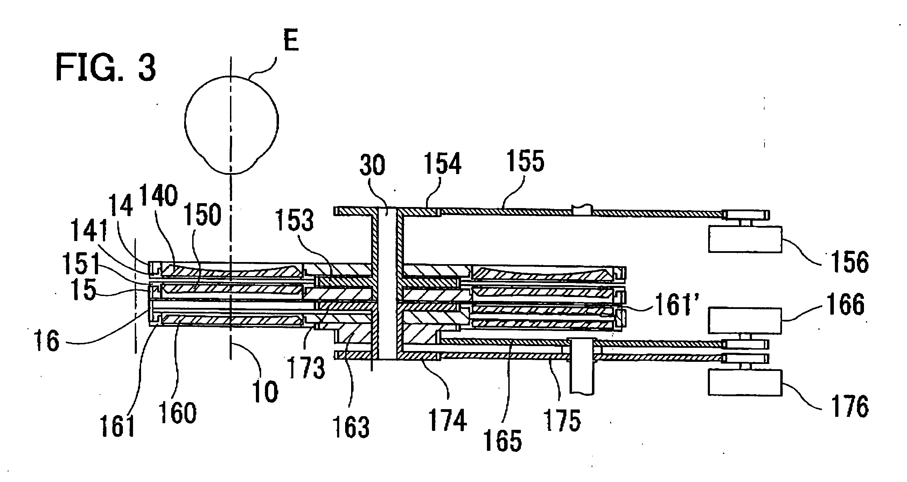

Fig. 3 is a schematic sectional view showing a rotation system of the cylindrical lenses 140 and 150 in the disks 14 and 15 respectively and the auxiliary lens 160 in the disk 16. The cylindrical lens 140 is set in the disk 14 with a holder 141 formed with gear teeth on the periphery so that the lens 140 is rotatable about the optical axis 10. Similarly, the cylindrical lens 150 is set in the disk 15 with a holder 151 formed with gear teeth on the periphery so that the lens 150 is rotatable about the optical axis 10. The gear teeth of the holders 140 and 150 both engage a sun gear 153 rotatable about the shaft 30. Accordingly, the rotation of a motor 156 is transmitted to the cylindrical lenses 140 and the 150 at the same time through a relay gear 155 and a gear 154 connected with the sun gear 153.

The auxiliary lens 160 is set in the disk 16 with a holder 161 formed with gear teeth on the periphery so that the lens 160 is rotatable about the optical axis 10. The gear teeth of the holder 161 engages a sun gear 163 rotatable about the shaft 30. Accordingly, the rotation of a motor 166 is transmitted to the auxiliary lens 160 through a relay gear 165 and a gear integrally formed in the sun gear 163. In the case where the rotary prism is rotatably set in the disk 16, an additional holder 161' is attached to the disk 16 so that two prisms are held in one hole to be rotatable about the optical axis 10. This additional holder 161' also has a periphery formed with gear teeth which engage a sun gear 173. Accordingly, the rotation of a motor 176 is transmitted to the rotary prism through a relay gear 175 and a gear 174 connected with the sun gear 173.

As explained above, the optical elements in the disks 14, 15, and 16 placed at positions farther from the examinee's eye E are designed to have a larger diameter (effective diameter) than the optical elements in the disks 11, 12, and 13 disposed at positions nearer the eye E. Thus, the view angle of the eye E can be widened. The view angle α of the eye E is determined based on an effective diameter φ of the auxiliary lens 160 in the disk 16 at a position farthest from the eye E and a lens distance *d* from the spherical lens 110 in the disk 11 at a position nearest the eye E to the auxiliary lens 160 in the disk 16 at the position farthest from the eye E. Herein, a distance VD, along the optical axis 10, between the corneal apex of the eye E and the surface of the spherical lens 110 facing the eye E in the disk 11 placed at the position nearest the eye E is set at a predetermined distance. The VD is set at about 12 mm in most cases in Japan. Assuming that the effective diameter φ of the auxiliary lens 160 is about 35 mm and the lens distance *d* is about 28 mm, a large view angle α of about 46° can be provided (see Fig. 2). In this case, the protective glass 40a in the test window 4 on the examiner side is correspondingly designed to have a diameter (an effective diameter) larger than the auxiliary lens 160 at the position farthest from the eye E to provide the above view angle α. Increasing the view angle α can reduce the influence of the instrument myopia on examination results. Consequently, correction powers (values) of spherical power S, cylindrical (astigmatic) power C, and astigmatic axis angle A can be measured with accuracy. It is generally preferable to provide a view angle α of at least about 40°. To provide the view angle α of about 40° when the distance VD is about 12 mm and the distance *d* is about 28 mm, the effective diameter φ of the optical element in the disk placed at the position farthest from the eye E is set at about 30 mm or more.

In the above case, the optical element to be arranged at the position nearest the eye E is preferably a spherical lens. Additionally, the cylindrical lens to be arranged at the position farther than the spherical lens is preferably a lens having a larger diameter (effective diameter) than the spherical lens in order to provide an adequate view angle α. Preferably, the diameter (the effective diameter) of the cylindrical lens is larger by about 10 mm or more than that of the spherical lens. In the present embodiment, the cylindrical lenses 140 and 150 are both about 36 mm in diameter (about 35 mm in effective diameter) as with the auxiliary lens 160 arranged at the position farthest from the eye E. However, since the spherical lens 110 is about 20 mm in effective diameter, the above view angle α of about 46° can be provided even where each cylindrical lens 140 and 150 is about 30 mm in effective diameter.

Further, the disk 16 may be removed. In this case, the distance *d* is defined as a distance between the spherical lens 110 in the disk 11 and the cylindrical lens 150 in the disk 15, providing an increased view angle α. Assuming that the effective diameter φ of the cylindrical lens 150 is about 35 mm and the lens distance *d* is about 21 mm, a view angle α of about 55° can be provided. To provide the view angle of about 46°, accordingly, the cylindrical lens 150 is required only to be about 30 mm in effective diameter φ.

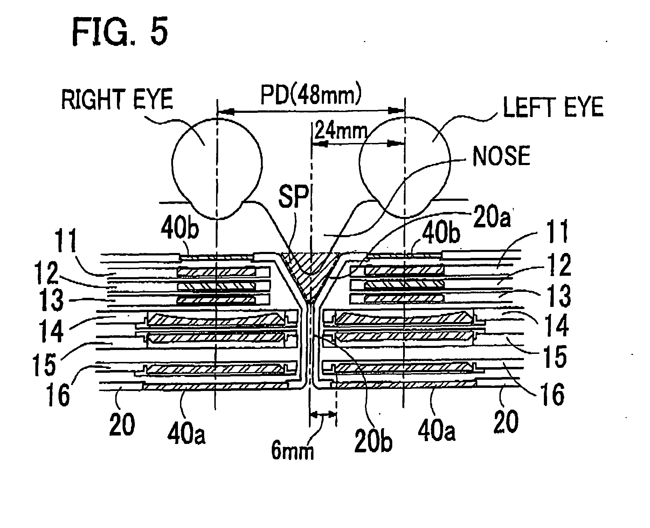

In the eye examination, the right and left lens chamber units 2 are moved to adjust the center distance between the right and left test windows 4 to the pupillary distance of the examinee. When the center distance is adjusted to a minimum specification (for example, about 48 mm) of the pupillary distance PD, it is necessary to prevent the covers 20 of the right and left lens chamber units 2 from interfering with each other. Such interference of the covers 20 can be avoided when each center point of the windows 4 is positioned on an imaginary line connecting the rotation centers of the disks (i.e., the center of the shaft 30) placed in the right and left lens chamber units 2 respectively. For example, if the pupillary distance PD is about 48 mm, the distance from the center of the examinee's eye to the center line of the examinee's nose is about 24 mm. Thus, if the maximum diameter of an optical element set in the disk is about 36 mm, a distance between the peripheral edge of the optical element and the center line of the nose (or the center line between the lens chamber units 2) is 6 mm (given by the expression; 24 - 36/2 = 6). In this case, accordingly, the corresponding holder, the corresponding disk, and the cover 20 of the lens chamber unit 2 have to be constructed to hold the optical element so that their edges are positioned within 6 mm (see Fig. 5).

Moreover, the spherical lens and other lenses 110-130 in the disks 11-13 arranged nearer the eye E are about 20 mm in diameter equal to the lenses in the conventional apparatus. On the other hand, the cylindrical lens and other lenses 140-160 in the disks 14-16 arranged farther from the eye E are about 36 mm in diameter larger than the lenses in the conventional apparatus, and accordingly, the disks 14-16 are designed to have a larger diameter. At this time, if the disks 11-13 and the disks 14-16 are equal in diameter, the covers 20 will more likely to interfere with the nose of the examinee. It is to be noted that most conventional apparatuses have been constructed such that each center point of the right and left test windows is positioned below the imaginary line connecting the rotation centers of the disks in order to avoid the interference of the covers of the right and left lens chamber units with the examiner's nose.

In the present embodiment, on the other hand, the disks 11, 12, and 13 each holding the small-diameter optical elements are adapted to be smaller in diameter than the disks 14, 15, and 16 each holding the large-diameter optical elements. Accordingly, the cover 20 is designed to have a side wall on the examinee's nose side being shorter in width in a direction of the interpupillary distance than an opposite side wall to correspond to the diameter difference, forming a concave portion SP serving as a space for examinee's nose, whereby preventing the covers 20 interfering with the nose. More specifically, as shown in Fig. 5, the cover 20 covering the small-diameter disks 11-13 placed nearer the examinee's nose is formed with a beveled end wall portion 20a and a straight end wall portion 20b covering the large-diameter disks 14-16. The beveled portion 20a slants in a direction that comes away from the center of the covers 20 with respect to the straight portion 20b. The beveled portions 20a can provide in combination the concave portion SP in which the examinee's nose is allowed to be positioned.

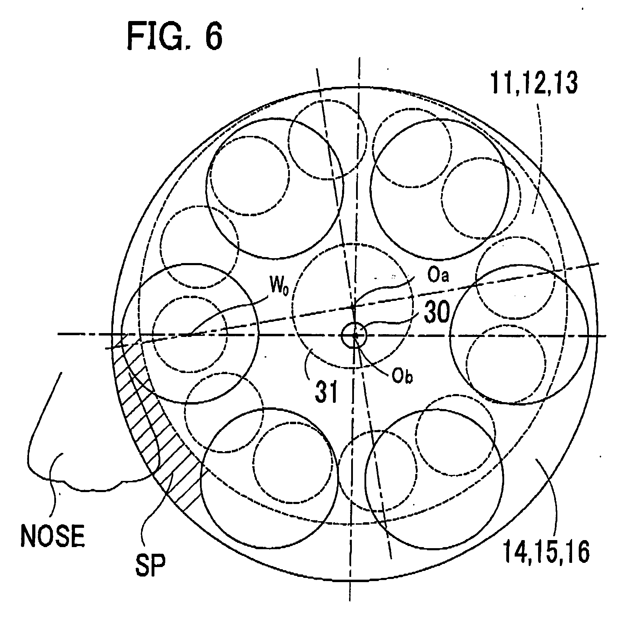

As shown in Fig. 6, furthermore, when the small-diameter disks 11, 12, and 13 are arranged eccentrically to the large-diameter disks 14, 15, and 16 with reference to the center Wo of the test window 4 such that the rotation center Oa of the small-diameter disks 11-13 is positioned above the rotation center Ob of the large-diameter disks 14-16, the concave portion SP for examinee's nose can be widened. Accordingly, a shaft 31 serving as the rotation center of the small-diameter disks 11, 12, and 13 is placed eccentrically to the shaft 30 serving as the rotation center of the large-diameter disks 14, 15, and 16.

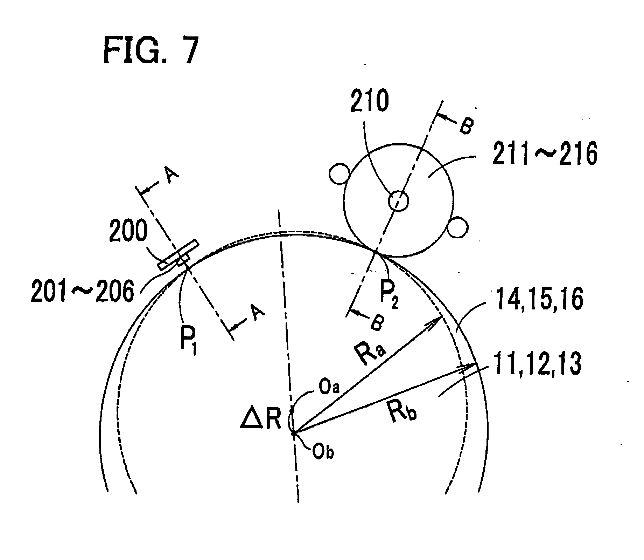

In addition, as shown in Fig. 7, an eccentric amount ΔR between the small-diameter disks 11, 12, and 13 and the large-diameter disks 14, 15, and 16 (namely, a distance between the rotation center Oa of the small-diameter disks 11-13 and the rotation center Ob of the large-diameter disks 14-16) is preferably determined to be larger than a difference between the radius Rb of each large-diameter disk 14, 15, and 16 and the radius Ra of each small-diameter disk 11, 12, and 13. Accordingly, the peripheral edges of the small-diameter and large-diameter disks overlap at two points (P1 and P2), leading to the following advantages.

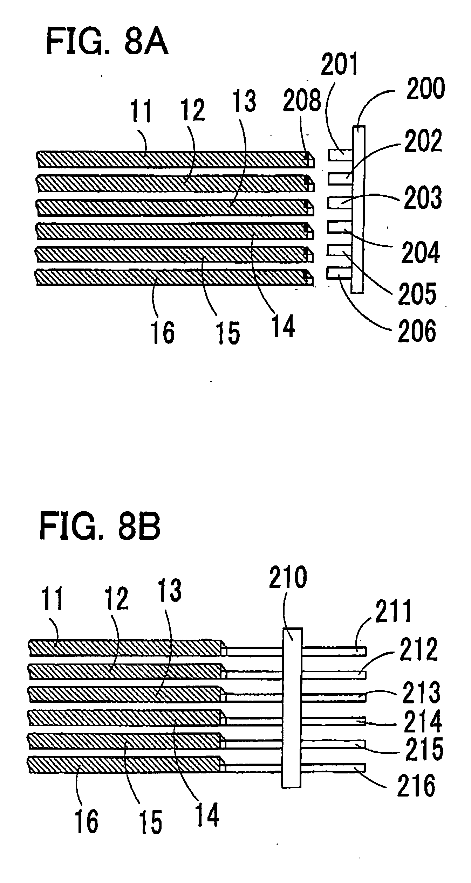

Fig. 8A is a sectional view at a point P1, taken along a line A-A in Fig. 7. At a position facing the point P1, reflective sensors 201-206 are placed in line on a base board 200 to detect each initial position of the disks 11-16. This structure has advantages in cost and installation space of the sensors. It is to be noted that the periphery of each disk 11-16 is attached, at a predetermined portion, with a light reflector 208 which is detected by the corresponding sensor 201-206.

Fig. 8B is a sectional view at a point P2, taken along a line B-B in Fig. 7. In contact with the point P2, relay gears 211-216 of the same diameter (gear ratio) are placed rotatably about a shaft 210 to rotate the different-diameter disks 11-16. The relay gears 211-216 are connected with the motors 18a-18f. Such placement of the relay gears 211-216 will effect a saving in arrangement space and also a reduction in number and standardization of components, resulting in a cost advantage. If the relay gears 211-216 are hard to arrange all together due to the sizes of the motors 18a-18f, the apparatus may be constructed so that only four relay gears 211-214 are attached rotatably about the shaft 210.

It is to be noted that, in the above explanation, the present invention is applied to the motor-driven optometric apparatus. As an alternative design, the present invention may be applied to an optometric apparatus constructed such that each disk and each optical element are manually rotated by means of a knob or the like.

As explained above, according to the present invention, an examination can be performed with high accuracy and little influence of intervention of accommodation. In addition, the right and left lens chamber units can be prevented from interfering with the examinee's nose.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An optometric apparatus (1) for subjectively examining a refractive power of an eye of an examinee, the apparatus including:
a pair of lens chamber units (2) each having a test window (4), one for a right eye of the examinee and the other for a left eye;
rotary disks (11-16) in each of which a plurality of optical elements (110-160) are set, the disks being rotatably placed in each lens chamber unit and including first rotary disks (11-13) placed at positions nearer the eye and second rotary disks (14-16) placed at positions farther from the eye;
wherein the optical elements (140-160) in the second rotary disks are larger in effective diameter than the optical elements (110-130) in the first rotary disks.

2. The optometric apparatus according to claim 1, wherein optical elements (160) set in a rotary disk (16) placed at the position farthest from the eye among the second rotary disks (14-16) have an effective diameter determined to provide a view angle of about 40° or more.

3. The optometric apparatus according to claim 1 or 2, wherein optical elements (160) set in a rotary disk (16) placed at the position farthest from the eye among the second rotary disks (14-16) have an effective diameter of about 30 mm or more.

4. The optometric apparatus according to any one of claims 1 to 3, wherein the optical elements (110-130) in the first rotary disks (11-13) each have an effective diameter of about 19 mm, and the optical elements (140-160) in the second rotary disks (14-16) each have an effective diameter of about 35 mm.

5. The optometric apparatus according to any one of claims 1 to 4, wherein the first rotary disks (11-13) include a rotary disk (11, 12) in which a spherical lens (110, 120) is set as the optical element, and the second rotary disks (14-16) include a rotary disk (14, 15) in which a cylindrical lens (140, 150) is set as the optical element.

6. The optometric apparatus according to claim 5, wherein the cylindrical lens (140, 150) has an effective diameter which is larger by about 10 mm or more than the spherical lens.

7. The optometric apparatus according to any one of claims 1 to 6, wherein the first rotary disks (11-13) have a smaller diameter than the second rotary disks (14-16).

8. The optometric apparatus according to claim 7, wherein the first rotary disks (11-13) are eccentrically arranged to the second rotary disks (14-16).

9. The optometric apparatus according to claim 8, wherein an eccentric amount between the first rotary disks (11-13) and the second rotary disks (14-16) is larger than a difference between a radius of the first rotary disks (11-13) and a radius of the second rotary disks (14-16).

10. The optometric apparatus according to any one of claims 7 to 9, wherein the right and left lens chamber units each includes a portion formed to provide in combination a concave portion for preventing interference of each unit with an examinee's nose.
